(19) **Europäisches Patentamt / European Patent Office / Office européen des brevets**

(11) **EP 2 774 980 B1**

(12) **EUROPEAN PATENT SPECIFICATION**

(45) Date of publication and mention
of the grant of the patent:
**04.01.2017 Bulletin 2017/01**

(21) Application number: **12846026.8**

(22) Date of filing: **24.10.2012**

(51) Int Cl.:
*C12N 1/16* (2006.01)     *C12P 7/08* (2006.01)
*C12P 7/06* (2006.01)     *C12P 7/10* (2006.01)
*C12R 1/72* (2006.01)

(86) International application number:
**PCT/JP2012/077428**

(87) International publication number:
**WO 2013/065541 (10.05.2013 Gazette 2013/19)**

(54) **NOVEL YEAST AND METHOD FOR PRODUCING ETHANOL USING SAME**

NEUARTIGE HEFE UND VERFAHREN ZUR ETHANOLHERSTELLUNG DAMIT

NOUVELLE LEVURE ET PROCÉDÉ DE PRODUCTION D'ÉTHANOL L'UTILISANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.11.2011 JP 2011240158**

(43) Date of publication of application:
**10.09.2014 Bulletin 2014/37**

(73) Proprietor: **Cosmo Oil Co., Ltd.
Tokyo 105-8528 (JP)**

(72) Inventor: **NAGASAKI, Hiroshi
Satte-shi
Saitama 340-0193 (JP)**

(74) Representative: **Blodig, Wolfgang
Wächtershäuser & Hartz
Patentanwaltspartnerschaft mbB
Weinstrasse 8
80333 München (DE)**

(56) References cited:
**JP-A- S5 828 289     US-A- 4 472 501**

- **LEANDRO MARIA JOSE ET AL: "Two glucose/xylose transporter genes from the yeast Candida intermedia: first molecular characterization of a yeast xylose-H+ symporter", BIOCHEMICAL JOURNAL, PUBLISHED BY PORTLAND PRESS ON BEHALF OF THE BIOCHEMICAL SOCIETY, vol. 395, no. Part 3, 1 May 2006 (2006-05-01), pages 543-549, XP002431284, ISSN: 0264-6021, DOI: 10.1042/BJ20051465**
- **"Candida intermedia NBRC 10601", National Institute of Tecnology and Evaluation, NTE Biological Resource Center (NBRC) , 27 April 2015 (2015-04-27), XP002738977, Retrieved from the Internet: URL:http://www.nbrc.nite.go.jp/NBRC2/NBRCCatalogueDetailServlet?ID=NBRC&CAT=00010601 [retrieved on 2015-04-27]**
- **"Kluyveromyces cellobiovorus", CBS Database, CBS-KNAW Fungal Biodiversity Centre , 27 April 2015 (2015-04-27), XP002738978, Retrieved from the Internet: URL:http://www.cbs.knaw.nl/Collections/BioloMICS.aspx?Link=T&TableKey=14682616000000 005&Rec=1001681&Fields=All [retrieved on 2015-04-27]**
- **MORIKAWA, Y. ET AL.: 'Ethanol productions from D-xylose and cellobiose by Kluyveromyces cellobiovorus' BIOTECHNOLOGY AND BIOENGINEERING vol. 27, no. 4, 1985, pages 509 - 13, XP055067468**

**(Cont. next page)**

• GARDONYI,M. ET AL.: 'High capacity xylose transport in Candida intermedia PYCC 4715' FEMS YEAST RESEARCH vol. 3, no. 1, 2003, pages 45 - 52, XP002431281
• NIDETZKY,B. ET AL.: 'Multiple Forms of Xylose Reductase in Candida intermedia: Comparison of Their Functional Properties Using Quantitative Structure-Activity Relationships, Steady-State Kinetic Analysis, and pH Studies' J AGRIC FOOD CHEM vol. 51, no. 27, 2003, pages 7930 - 7935, XP055067471
• RAJESHAM,S.: 'Ethanol production from D-xylose by Candida intermedia MTCC-1404: Parameter optimization using Taguchi's overall evaluation criteria technique' ASIAN JOURNAL OF MICROBIOLOGY, BIOTECHNOLOGY AND ENVIRONMENTAL SCIENCES vol. 7, no. 4, 2005, pages 679 - 684, XP008173321
• VENKATESWAR,R.L.: 'Isolation and screening of yeast for ethanol and xylitol production from D-xylose' ASIAN JOURNAL OF MICROBIOLOGY, BIOTECHNOLOGY AND ENVIRONMENTAL SCIENCES vol. 8, no. 4, 2006, pages 785 - 789, XP008173320

**Description**

[Technical Field]

The present invention relates to a novel yeast and a method for producing ethanol using the same.

[Background Art]

In recent years, large-scale production of bioethanol has been conducted throughout the world as a countermeasure against global warming. A main raw material for bioethanol is edible biomass such as corn biomass or sugarcane biomass. Such edible biomass is problematic in terms of a competition between the use as a raw material for bioethanol and the use as a food material.

In order to avoid such a problem, it has been desired to develop a technique of producing ethanol from a cellulosic biomass of wood, non-edible herb and the like, which are not used as food materials (particularly, cellulosic biomass obtained from agricultural residues, forestry residues, etc.).

In such a technique, cellulose or hemicellulose which is comprised in cellulosic biomass, or a polysaccharide as a partially decomposed product of such cellulose or hemicellulose, is hydrolyzed to obtain a saccharified solution comprising, as main ingredients, hexose (glucose, mannose and galactose) and pentose (xylose). Subsequently, sugar(s) comprised in the saccharified solution are fermented by microorganisms, so as to obtain ethanol.

Saccharomyces cerevisiae is known as a yeast capable of efficiently producing ethanol from glucose and mannose. However, there are only several types of microorganisms, which are capable of efficiently producing ethanol from xylose or galactose.

For instance, Pichia stipitis, Candida shehatae and Pachysolen tannophilus (Non Patent Literature 1), and Candida intermedia (Non Patent Literature 2), are known as a few examples of microorganisms capable of producing ethanol not only from glucose but also from xylose.

Candida intermedia MTCC-1404 produces both ethanol (70%) and xylitol (11%) from D-xylose (Non-Paten Literature 3). Different carbon sources have an effect for the production of ethanol form D-xylose, e.g in the presence of glucose ethanol yield is greatly decreased (40%).

[Citation List]

[Non Patent Literature]

[Non Patent Literature 1]
Yablochkova, EN., Bolotnikova, OI., Mikhailova, NP., Nemova, NN. And Ginak, AI. Applied Biochemistry and Microbiology, Vol. 39, 302-306 (2003)
[Non Patent Literature 2]
Y. Morikawa, et al., Biotechnology andBioengineering, Vol: XXVII, 509-513 (1984)
[Non Patent Literature 3]
CH. Pavana Jyothi, et al., Asian Journal of Microbial Biotechnology and Environmental Sciences, Vol 8, N0.4, 785-789 (2006)

[Summary of Invention]

[Problem to be solved by the Invention]

However, the yeast described in Non Patent Literature 1 has been problematic in that, when ethanol fermentation is carried out in the presence of both glucose and xylose comprised in a raw material liquid derived from cellulosic biomass, almost no xylose is consumed until glucose has been almost completely consumed as a result of catabolite repression caused by glucose, and thus that a long period of time is required for fermentation, and ethanol productivity is thereby lowered.

The present inventor conducted studies regarding the yeast described in Non Patent Literature 2. As a result, it was found that catabolite repression is hardly caused by glucose, depending on production conditions, and that although there may be a case in which ethanol fermentation can be carried out from both glucose and xylose when it is carried out in the presence of the glucose and the xylose, the yeast described in Non Patent Literature 2 is problematic in that xylose consumption efficiency is poor, and in that the yeast described in Non Patent Literature 2 does not have a sufficient ability to produce ethanol from xylose.

Therefore, the present invention relates to provision of a novel yeast having an ability to efficiently produce ethanol from glucose and xylose in a short time in the coexistence of the glucose and the xylose and a method for producing ethanol using the novel yeast.

[Solution to Problem]

Hence, as a result of intensive studies, the present inventor found that a specific yeast has an ability to efficiently produce ethanol from glucose and xylose in a short time in the coexistence of the glucose and the xylose, thereby completing the present invention.

Specifically, the present invention provides a yeast, which was designated as Candida intermedia 4-6-4T2 and was deposited as FERM BP-11509.

In addition, the present invention also provides a method for producing ethanol, which comprises a step of fermenting a raw material liquid comprising one or more monosaccharides selected from glucose and xylose using the aforementioned yeast.

[Effects of Invention]

The yeast of the present invention has an ability to efficiently produce ethanol from glucose and xylose. in a short time in the coexistence of the glucose and the xylose. Moreover, using such a yeast, ethanol can be efficiently produced in a short time even in the case of using a raw material derived from cellulosic biomass comprising glucose or xylose.

Therefore, according to the method for producing ethanol of the present invention, ethanol can be efficiently produced in a short time from a cellulosic biomass-derived raw material.

[Brief Description of Drawings]

[Figure 1] Figure 1 is a view showing a change over time in individual ingredients when glucose and xylose are fermented with a parent strain.

[Figure 2] Figure 2 is a view showing a change over time in individual ingredients when glucose and xylose are fermented with a 4-6-4T2 strain.

[Figure 3] Figure 3 is a view showing a change over time in individual ingredients when xylose is fermented with a parent strain.

[Figure 4] Figure 4 is a view showing a change over time in individual ingredients when xylose is fermented with a 4-6-4T2 strain.

[Description of Embodiments]

<Yeast>

The yeast of the present invention is a yeast, which was designated as Candida intermedia 4-6-4T2 and was deposited at the International Patent Organism Depositary (IPOD), National Institute of Technology and Evaluation (NITE), Incorporated Administrative Agency (address: Tsukuba Central 6, Higashi 1-1-1, Tsukuba, Ibaraki, Japan, postal code: 305-8566) under accession No. FERM BP-11509 on September 20, 2012. This yeast was obtained by subjecting Candida intermedia "NBRC10601" used as a parent strain to spontaneous mutation according to an ordinary method and selecting a strain having a higher ethanol production ability than the parent strain. It is to be noted that the aforementioned "NBRC10601" is a yeast available from the International Patent Organism Depositary (IPOD), National Institute of Technology and Evaluation (NITE), Incorporated Administrative Agency.

The yeast of the present invention has an ability to efficiently produce ethanol from glucose and xylose in a short time in the coexistence of the glucose and the xylose.

Herein, the phrase "in the coexistence of glucose and xylose" is used to mean that the yeast of the present invention coexists in a raw material liquid (fermentation liquor) comprising at least glucose and xylose. As mentioned above, the conventional yeast has not had sufficient xylose consumption efficiency, or it has had an ability to produce ethanol from either one of glucose and xylose. In the case of the conventional yeast, when both glucose and xylose were present, almost no xylose was consumed, until glucose had been completely consumed by catabolite repression. In contrast, the yeast of the present invention has an ability to efficiently produce ethanol from both glucose and xylose in a short time, even when both the glucose and the xylose are present.

Moreover, while the yeast of the present invention efficiently produces ethanol from a raw material liquid comprising glucose and xylose in a short time, it produces almost no xylitol as a by-product. Furthermore, the yeast of the present invention has the same properties as those of its parent strain, other than such an ability to produce ethanol from sugar(s).

<Method for Producing Ethanol>

The method for producing ethanol of the present invention is characterized in that it comprises a step of fermenting a raw material liquid comprising one or more monosaccharides selected from glucose and xylose., using the aforementioned yeast (hereinafter also referred to as a "fermentation step"). According to this production method, ethanol can be efficiently produced in a short time.

The amount of the yeast used is not particularly limited. The yeast is used at a ratio of generally approximately from 0.01 to 100 mass parts, and preferably from 0.1 to 10 mass parts, based on 1 mass part of the above-mentioned monosaccharide(s).

The above-mentioned raw material liquid (for example, a saccharified solution) preferably comprises glucose and xylose. Moreover, in addition to these monosaccharides, the raw material liquid may also comprise other monosaccharides (hexoses) such as mannose or galactose. The production method of the present invention is hardly affected by catabolite repression. Hence, according to the present production method, even if the raw material liquid comprises the aforementioned monosaccharides (xylose, mannose, or galactose) as well as glucose, ethanol can be efficiently produced.

When the above-mentioned raw material liquid comprises glucose and xylose, with regard to the content ratios of glucose and xylose, xylose is used at a ratio of preferably 0.1 to 5 mass parts, and more preferably 0.5 to 3 mass parts, based on 1 mass part of glucose. Using the yeast of the present invention, even if the content ratios of glucose and xylose are within the above described ranges, ethanol can be produced not only from glucose but also from xylose. Moreover, each of the contents of glucose and xylose in the raw material liquid is preferably 1 to 100 g/L, and more preferably 5 to 50 g/L. Using the yeast of the present invention, even if each of the contents of glucose and xylose is within the above-described ranges, ethanol can be produced. Furthermore, the content ratio of glucose with respect to sugar(s) comprised in the raw material liquid is not particularly limited. Glucose is contained at a mass percentage of preferably 5% to 99 %, and more preferably 10% to 70 %, based on the total mass of the sugar(s) comprised in the raw material liquid.

The total content of monosaccharides comprised in the above described raw material liquid is preferably 1 to 100 g/L.

The raw material liquid used in ethanol production may be any one of a raw material liquid derived from animals, a raw material liquid derived from plants, and a raw material liquid derived from industrial wastes. Among these, a raw material liquid derived from plants is preferable. From the viewpoint of preventing a competition between the use as a raw material for bioethanol and the use as a food material, a cellulosic biomass hydrolysate is more preferable. The term "cellulosic biomass" is used herein to mean biomass comprising cellulose and hemicellulose. Glucose is obtained by hydrolyzing cellulose comprised in such biomass, whereas glucose, xylose, mannose, and galactose are obtained by hydrolyzing hemicellulose comprised therein.

From the viewpoint of economic advantage in ethanol production, such cellulosic biomass is preferably obtained from agricultural residues (rice straw, wheat straw, etc.), forestry residues (lumbers, etc.), and the like.

The pH (30°C) of the above described raw material liquid is preferably 3.5 to 6.5, more preferably 4 to 6, further preferably 4.5 to 5.5, and particularly preferably 4.5 to 5. According to the yeast of the present invention, ethanol production is possible even in such a low pH range. Thus, even in the case of using a saccharified solution comprising acetic acid or the like (an acid hydrolysate of biomass cellulose), ethanol can be produced.

It has been reported that a saccharified solution with a low pH value obtained by hydrolyzing cellulosic biomass using an acid comprises an inhibitory substances that inhibit ethanol production by microorganisms, such as acetic acid (Nigam JN. Journal of Applied Microbiology, Vol. 90, 208-215 (2001)). It has also been reported that, in particular, ethanol fermentation by microorganisms capable of producing ethanol from xylose is inhibited in a low pH range such as pH 5 or less (Palmqvist E., Hahn-Hagardal B. Bioresource Technology Vol. 74, 25-33 (2000)).

The above described production method is carried out at a temperature of preferably 20°C to 35°C, and more preferably 25°C to 30°C.

Moreover, in the above described production method, while the above described yeast may be allowed to grow under its growing conditions, ethanol may be produced. Alternatively, the yeast may be left in the state of resting cells, namely, the yeast may be left under conditions in which a nitrogen source is reduced to an amount insufficient for the growth of the yeast and a carbon source used as a raw material for ethanol is abundant, so that ethanol can be produced under conditions in which the growth of the yeast is suspended. Among these conditions, it is preferable to produce ethanol by the yeast which is in the state of resting cells, since ethanol production is hardly affected by ethanol production inhibitory substances, such as acetic acid or sulfurous acid, under such conditions. When ethanol production is carried out by the yeast which is in the state of resting cells, the concentration of the yeast is preferably 5 to 100 g/L. Moreover, when ethanol is produced by the yeast which is in the state of resting cells, it is not necessary to add a nitrogen source, a yeast extract, etc. to the raw material liquid. It is preferable that the raw material liquid be adjusted to have the aforementioned pH value by addition of a phosphate buffer, sodium hydroxide or the like. The recovery of ethanol may be carried out according to ordinary means such as distillation.

Furthermore, in the above described production method, from the viewpoint of the amount of cells, it is preferable that a pre-culture be carried out before the aforementioned fermentation step. As a medium used in such a pre-culture, a medium comprising glucose and one or more sugars selected from mannose, galactose and xylose is preferable. As such a medium, a medium comprising the aforementioned cellulosic biomass hydrolysate may be used.

The concentration of the aforementioned sugars in total is preferably 1 to 100 g/L, and more preferably 10 to 50 g/L. The content ratio of one or more sugars selected from mannose, galactose and xylose to glucose is not particularly limited.

When a cellulosic biomass hydrolysate is used as a carbon source in the above described pre-culture, the hydrolysate is used at a volume percentage of generally 20% or less, and preferably 10% or less, based on the volume of the medium. Other ingredients comprised in the medium are not particularly limited. Examples of such other ingredients include: nitrogen sources suitable for growth, such as amino acid, urea, polypeptone, and an amino acid-free nitrogen base; and a yeast extract. The temperature applied to the pre-culture is preferably 10°C to 37°C, and more preferably 25°C to 30°C. The pH applied to the pre-culture is preferably 4 to 7, and more preferably 4.5 to 6.5. In addition, the pre-culture is preferably carried out under aerobic conditions.

[Examples]

Hereinafter, the present invention will be described in detail in the following examples.

Example 1

In accordance with the following procedures, the yeast Candida intermedia "NBRC10601," which was deposited at the International Patent Organism Depositary (IPOD), National Institute of Technology and Evaluation (NITE), Incorporated Administrative Agency, was used as a parent strain, and the yeast Candida intermedia "NBRC10601" was subjected to acclimation and spontaneous mutation, thereby obtaining a yeast strain 4-6-4T2.

First, the pH of an acetic acid aqueous solution comprising glucose and xylose (each 1 mass %) was adjusted to be 5.0 with magnesium hydroxide, and 20% of this solution was then mixed with 80% of a liquid medium (1% yeast extract, 2% amino acid-free nitrogen base). Thereafter, 1% xylose was added to 10 mL of the obtained mixed solution, and one platinum loop of the yeast Candida intermedia "NBRC10601" was then inoculated into the mixed solution. The thus obtained mixture was cultured at 30°C for 3 days to obtain a culture solution.

Subsequently, in the same manner as described above, 50% of an acetic acid aqueous solution comprising glucose and xylose (each 1 mass %) that had been adjusted to have pH 5.0 was mixed with 50% of a liquid medium. Thereafter, to 10 mL of this mixed solution, 100 μL of the culture solution obtained as a result of the aforementioned culture for 3 days was added, and the thus obtained mixture was further cultured for 7 days. Thereafter, in the same manner as described above, 80% of an acetic acid aqueous solution comprising glucose and xylose (each 1 mass %) that had been adjusted to have pH 5.0 was mixed with 20% of a medium. To 10 mL of this mixed solution, 100 μL of the culture solution obtained as a result of the aforementioned culture for 7 days was added, and the obtained mixture was further cultured for 30 days, so as to prepare an acclimated strain solution.

The prepared acclimated strain solution was diluted by 1000 times, and the thus diluted solution was applied onto a YNB agar medium (5% glucose, 1% yeast extract, 2% amino acid-free nitrogen base, 2% agar), and the resultant was then cultured at 25°C for 4 days. Thereafter, a strain that formed a colony was obtained.

The obtained strain was applied onto a YNB agar medium (2% trehalose, 1% yeast extract, 2% amino acid-free nitrogen base, 2% agar), and the resultant was cultured 25°C for 3 days. Thereafter, formation of a colony was confirmed, and the culture was then stored at 4°C. Colonies, which had grown at 4°C, were selected, and thereafter, using the selected colonies, an ethanol production test was carried out in a phosphate buffer (2.5% xylose, 0.1 M $KH_2PO_4$, pH = 5.0, 0.006 M MgSO4·7H$_2$O). A strain having an ability to produce ethanol, which was higher than the parent strain, was selected.

Thus, a yeast of interest was selected, and was designated as Candida intermedia 4-6-4T2. The present yeast strain was deposited at the International Patent Organism Depositary (IPOD), National Institute of Technology and Evaluation (NITE), Incorporated Administrative Agency. The accession number is FERM BP-11509.

Example 2

The parent strain (NBRC10601) and the strain 4-6-4T2 were each inoculated into a YNB medium (0.5% glucose, 2% xylose, 1% yeast extract, 2% amino acid-free nitrogen base), and the obtained mixtures were each subjected to a shaking culture at 30°C at 120 rpm (24 to 48 hours, $OD_{660}$ = 18 to 25). The weight of a dry yeast strain in 1 mL of the culture solution was calculated according to the following relational expression (1), which was obtained empirically. (According to the expression (1), the weight of a dry yeast strain could be calculated to be 0.0062 g in the case of $OD_{660}$ = 20, for example.)

```
Weight of dry yeast strain (g/mL) = 0.00032 × OD₆₆₀ -

0.00017 ... (1)
```

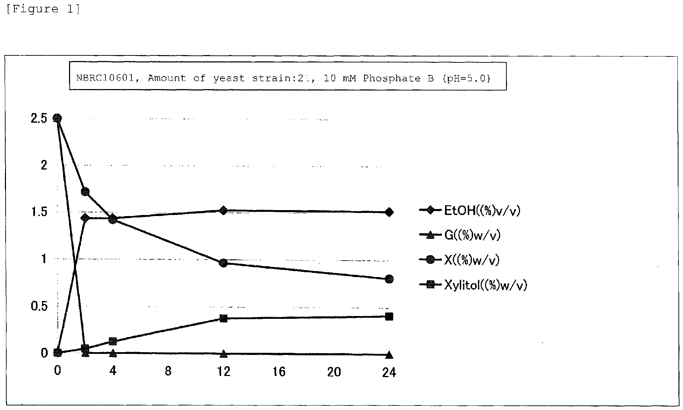

A necessary amount of culture solution was recovered by centrifugation (3000 rpm, 2 minutes) such that the amount of the yeast strain became 2% in terms of dry weight. Thereafter, a fermentation solution prepared by mixing the sugars shown in Tables 1 to 4 below (raw materials for ethanol) into a phosphate buffer ($KB_2PO_4/K_2HPO_4$: 0.01M, pH = 5.0) was added to each yeast strain, followed by performing fermentation. A change over time in the amount of ethanol produced was confirmed. The results are shown in Tables 1 to 4 and Figures 1 to 4. It is to be noted that the symbols G and X used in the tables and the figures indicate glucose and xylose, respectively.

[Table 1]

| Fermentation (h) | EtOH((%)v/v) | Xylitol((%)w/v) | G((%)w/v) | X((%)w/v) |
|---|---|---|---|---|
| 0 | 0 | 0 | 2.5 | 2.5 |
| 2 | 1.43 | 0.04 | 0 | 1.71 |
| 4 | 1.43 | 0.12 | 0 | 1.42 |
| 12 | 1.52 | 0.38 | 0 | 0.96 |
| 24 | 1.52 | 0.41 | 0 | 0.81 |
| Cell mass used: NBRC10601, Sugars: G (2.5%) + X (2.5%) | | | | |

[Table 2]

| Fermentation (h) | EtOH((%)v/v) | xylitol((%)w/v) | G((%)w/v) | X((%)w/v) |
|---|---|---|---|---|
| 0 | 0 | 0 | 2.5 | 2.5 |
| 2 | 1.04 | 0.09 | 0.44 | 2.00 |
| 4 | 1.60 | 0.16 | 0 | 1.48 |
| 12 | 1.96 | 0.20 | 0 | 0.15 |
| 24 | 2.14 | 0.22 | 0 | 0 |
| Cell mass used: 4-6-4T2, Sugars: G (2.5%) + X (2.5%) | | | | |

[Table 3]

| Fermentation (h) | EtOH((%)v/v) | xylitol((%)w/v) | X((%)w/v) |
|---|---|---|---|
| 0 | 0 | 0 | 5 |
| 2 | 0.25 | 0.25 | 3.35 |
| 4 | 0.28 | 0.38 | 2.95 |
| 12 | 0.57 | 0.44 | 2.13 |

(continued)

| Fermentation (h) | EtOH((%)v/v) | xylitol((%)w/v) | X((%)w/v) |
|---|---|---|---|
| 24 | 0.80 | 0.77 | 1.69 |
| Cell mass used: NBRC10601, Sugars: X (5%) | | | |

[Table 4]

| Fermentation (h) | EtOH((%)v/v) | xylitol((%)w/v) | X((%)w/v) |
|---|---|---|---|
| 0 | 0 | 0 | 5 |
| 2 | 0.43 | 0.06 | 3.25 |
| 4 | 0.81 | 0.06 | 2.16 |
| 12 | 1.42 | 0.13 | 0.81 |
| 24 | 1.75 | 0.15 | 0.23 |
| Cell mass used: 4-6-4T2, Sugars: X (5%) | | | |

As shown in Table 1 and Figure 1, when ethanol was produced by the parent strain (2%) using glucose and xylose (each 2.5%) as carbon sources, both the glucose and the xylose were consumed at the initial stage of fermentation and ethanol was produced. However, at the time at which all glucose was consumed, ethanol production was terminated, and also, consumption of xylose was almost terminated. From these results, it is found that the parent strain was hardly affected by catabolite repression caused by glucose, but that it had a low ability to produce ethanol from xylose.

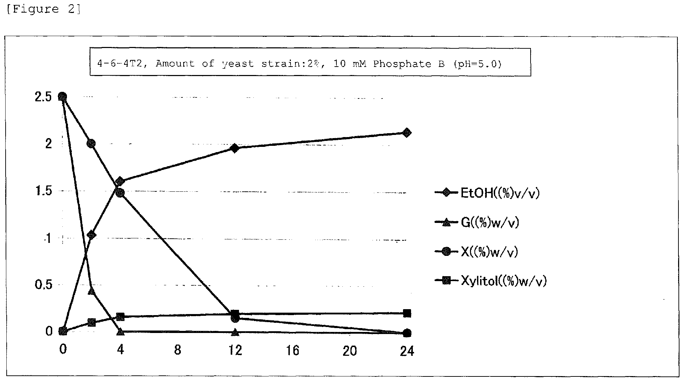

In contrast, as shown in Table 2 and Figure 2, when ethanol was produced by the strain 4-6-4T2 (2%) using glucose and xylose (each 2.5%) as carbon sources, both the glucose and the xylose were consumed at the initial stage of fermentation, as in the case of the parent strain. Not only ethanol was produced from both the glucose and the xylose, but also consumption of xylose progressed at a nearly constant rate even after completion of glucose consumption. As a result, not only glucose but also xylose was sufficiently consumed, and together with this phenomenon, the amount of ethanol produced was also increased. From these results, it is found that the strain 4-6-4T2 inherited the property of being hardly affected by catabolite repression caused by glucose from the parent strain, and at the same time, the strain 4-6-4T2 had an ability to produce ethanol from xylose that had been significantly improved when compared with the parent strain.

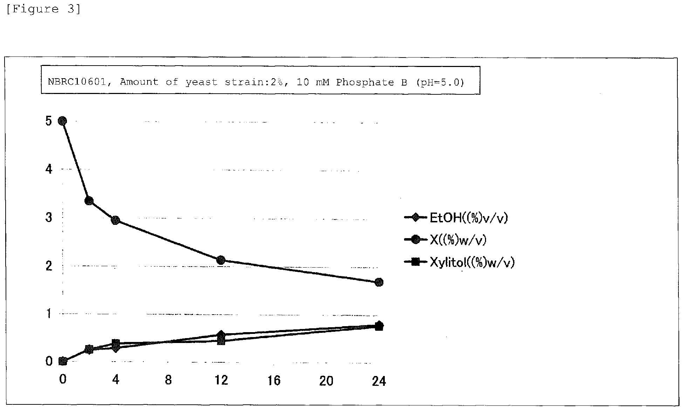

In addition, as shown in Table 3 and Figure 3, when ethanol was produced by the parent strain (2%) using xylose (5%) as a carbon source, 30% or more of xylose remained even after 24 hours of fermentation.

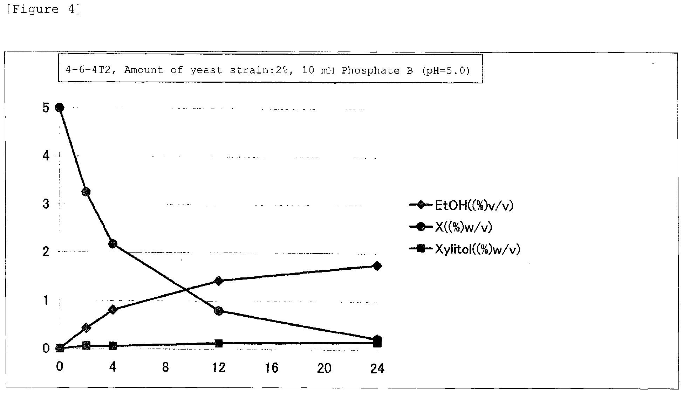

In contrast, as shown in Table 4 and Figure 4, when ethanol was produced by the strain 4-6-4T2 (2%) using xylose (5%) as a carbon source, 90% or more of xylose was consumed after 24 hours of fermentation, and ethanol was produced in an amount approximately 1.4 times higher than that in the case of using the parent strain.

The reason why there is a difference between the strain 4-6-4T2 and the parent strain in terms of ability to produce ethanol from xylose has not been necessarily clarified. From the above Figures 1 to 4, however, it is assumed that production of ethanol from xylose would be suppressed or inhibited by accumulation of xylitol which is an intermediate metabolite during the production of ethanol from xylose. That is to say, from the comparison between Figures 1 and 3 each showing a change over time in fermentation using the aforementioned parent strain, and Figures 2 and 4 each showing a change over time in fermentation using the aforementioned strain 4-6-4T2, it is considered that a large amount of xylitol is produced by the parent strain, and with such an increase in the amount of xylitol produced, consumption of xylose is lowered, and ethanol production tends to be terminated.

Thus, it is assumed that the reason that ethanol can be efficiently produced using the yeast of the present invention would be that the present yeast is hardly affected by catabolite repression caused by glucose, and also that production of xylitol is suppressed and thereby ethanol productivity is hardly inhibited by such xylitol.

Example 3

The yeasts shown in Table 5 (approximately 2% dry weight) were each added to a fermentation solution (0.01 M phosphate buffer, pH 5.0) each comprising the sugars shown in the same table. Twelve hours later, the amount of ethanol produced (% (v/v)) was determined. The results are shown in Table 5.

[Table 5]

| %(v/v) | 4-6-4T-2 | C. intermedia NBRC 10601 | S. cerevisiae NBRC 0216 | P. tannophilus ATCC32691 | C. shehatae ATCC22984 | P. stipits ATCC58785 |
|---|---|---|---|---|---|---|
| Xylose (2.5%) | 1.2 | 1.1 | 0.0 | 0.9 | 1.0 | 1.1 |
| Xylose (2.5%)+ + Glucose(2.5%) | 2.3 | 1.9 | 1.2 | 2.0 | 2.0 | 2.2 |
| Xylose(2.5%)+ Glucose(1.2%)+ Mannose(1.2%) | 2.6 | 2.4 | 1.2 | 2.0 | 2.0 | 2.2 |
| Xylose(2.5%)+ Glucose(1.2%)+ Galactose(1.2%) | 2.6 | 2.5 | 1.2 | 1.5 | 2.0 | 2.2 |
| Xylose(2.3%)+ Glucose(0.9%)+ Mannose(0.6%)+ Galactose(0.6%) | 2.4 | 2.1 | 1.2 | 1.5 | 2.0 | 2.2 |

From Table 5, it is found that the strain 4-6-4T2 exhibits an ability to produce ethanol, which is higher than that of the parent strain or known yeast strains, regardless of the types of sugars used.

**Claims**

1. A yeast, which was designated as Candida intermedia 4-6-4T2 and was deposited as FERM BP-11509.

2. A method for producing ethanol, which comprises a step of fermenting a raw material liquid comprising at least one monosaccharides selected from glucose and xylose, using the yeast according to claim 1.

3. The method according to claim 2, wherein the raw material liquid comprises glucose and xylose.

4. The method according to claim 2 or 3, wherein the pH of the raw material liquid is 3.5 to 6.5.

5. The method according to any one of claims 2 to 4, wherein a cellulosic biomass hydrolysate is used as the raw material liquid.

**Patentansprüche**

1. Eine Hefe, die als Candida intermedia 4-6-4T2 bezeichnet als FERM BP-11509 hinterlegt wurde.

2. Verfahren zur Herstellung von Ethanol, welches einen Schritt des Fermentieren einer Rohmaterialflüssigkeit, die mindestens ein Monosaccharid ausgewählt aus Glucose und Xylose umfasst, unter Verwendung der Hefe gemäß Anspruch 1 umfasst.

3. Verfahren nach Anspruch 2, wobei die Rohmaterialflüssigkeit Glucose und Xylose umfasst.

4. Verfahren nach Anspruch 2 oder 3, wobei der pH-Wert der Rohmaterialflüssigkeit 3,5 bis 6,5 ist.

5. Verfahren nach einem der Ansprüche 2 bis 4, wobei ein Zellulose-Biomasse-Hydrolysat als Rohmaterialflüssigkeit verwendet wird.

**Revendications**

1. Levure qui a été appelée Candida intermedia 4-6-4T2 et qui a été déposée sous la référence FERM BP-11509.

2. Procédé pour produire de l'éthanol, qui comprend une étape de fermentation d'une matière première liquide comprenant au moins un monosaccharide choisi parmi le glucose et le xylose, utilisant la levure selon la revendication 1.

3. Procédé selon la revendication 2, dans lequel la matière première liquide comprend du glucose et du xylose.

4. Procédé selon la revendication 2 ou 3, dans lequel le pH de la matière première liquide est de 3,5 à 6,5.

5. Procédé selon l'une quelconque des revendications 2 à 4, dans lequel un hydrolysat de biomasse cellulosique est utilisé en tant que matière première liquide.

[Figure 1]

[Figure 2]

[Figure 3]

[Figure 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YABLOCHKOVA, EN. ; BOLOTNIKOVA, OI. ; MIKHAILOVA, NP. ; NEMOVA, NN. ; GINAK, AI.** *Applied Biochemistry and Microbiology,* 2003, vol. 39, 302-306
- **Y. MORIKAWA et al.** *Biotechnology and Bioengineering,* 1984, vol. XXVII, 509-513
- **CH. PAVANA JYOTHI et al.** *Asian Journal of Microbial Biotechnology and Environmental Sciences,* 2006, vol. 8 (4), 785-789
- **NIGAM JN.** *Journal of Applied Microbiology,* 2001, vol. 90, 208-215
- **PALMQVIST E. ; HAHN-HAGARDAL B.** *Bioresource Technology,* 2000, vol. 74, 25-33